# FASCICULE DE BREVET EUROPEEN

(11) **EP 4 488 681 B1**
(45) Date de publication et mention de la délivrance du brevet: **02.09.2026**
(21) Numéro de dépôt: 24186921.3
(22) Date de dépôt: 05.07.2024
(51) Int. Cl.: G01N 33/483, G01N 21/64

(54) **DISPOSITIF D'IMAGERIE DE L'ACTIVITÉ ÉLECTRIQUE D'UN ÉCHANTILLON, SYSTÈME D'IMAGERIE, PROCÉDÉ DE D'ANALYSE D'UN ÉCHANTILLON ET PROCÉDÉ DE FABRICATION ASSOCIÉS**
VORRICHTUNG ZUR ABBILDUNG DER ELEKTRISCHEN AKTIVITÄT EINER PROBE, BILDGEBUNGSSYSTEM, VERFAHREN ZUR ANALYSE EINER PROBE UND HERSTELLUNGSVERFAHREN DAFÜR
DEVICE FOR IMAGING THE ELECTRICAL ACTIVITY OF A SAMPLE, IMAGING SYSTEM, METHOD FOR ANALYSING A SAMPLE AND ASSOCIATED MANUFACTURING METHOD

(30) Priorité: 07.07.2023 FR 2307290
(43) Date de publication de la demande: 08.01.2025
(73) Titulaire: Commissariat à l'Energie Atomique et aux Energies Alternatives, 75015 Paris (FR)
(72) Inventeur: HAON, Olivier, 38054 GRENOBLE CEDEX 09 (FR); RACINE, Benoit, 38054 GRENOBLE CEDEX 09 (FR)
(74) Mandataire: Cabinet Camus Lebkiri

(56) Documents cités:
- US-A1- 2015 261 024
- WEE DONGHO ET AL: "Thermal-induced optical modulation of liquid crystal embedded polymer film on silver nanowire heater", SOLAR ENERGY MATERIALS AND SOLAR CELLS, ELSEVIER SCIENCE PUBLISHERS, AMSTERDAM, NL, vol. 147, 24 December 2015 (2015-12-24), pages 150 - 156, XP029397258, ISSN: 0927-0248, DOI: 10.1016/J.SOLMAT.2015.12.007
- TAKASHI TOKUDA ET AL: "Optical and Electric Multifunctional CMOS Image Sensors for On-Chip Biosensing Applications", MATERIALS, vol. 4, no. 1, 29 December 2010 (2010-12-29), pages 84 - 102, XP055347233, DOI: 10.3390/ma4010084

## Description

### DOMAINE TECHNIQUE DE L'INVENTION

Le domaine technique de l'invention est lié à l'analyse d'un échantillon, par exemple un échantillon biologique, par un système d'imagerie.

L'invention concerne un dispositif d'imagerie destiné à l'analyse d'un échantillon, un système d'imagerie, un procédé d'analyse d'un échantillon et un procédé de fabrication du dispositif d'imagerie.

L'invention trouve une application dans les domaines de la biologie et de la santé, en particulier dans le domaine des organes sur puces, ou « organ-on-chip » en anglais.

### ARRIERE-PLAN TECHNOLOGIQUE DE L'INVENTION

Dans le domaine des neurosciences et de la médecine, la capacité à détecter, visualiser et enregistrer l'activité électrique des neurones est clé pour comprendre le fonctionnement et les dysfonctionnements du cerveau, et pour voir directement les effets de médicaments ou autres stimulations sur le fonctionnement d'une population de neurones.

Une application particulièrement importante concerne la compréhension de maladies neuro-dégénératives, telles que la maladie de Parkinson ou la maladie d'Alzheimer, ainsi que le développement de médicaments contre ces maladies.

Les techniques d'imagerie électrophysiologique reposent généralement sur une approche de détection/transduction électronique.

On trouve ainsi des matrices de microélectrodes disponibles commercialement, également appelées MEAs (acronyme de Micro-Electrode Arrays https://en.wikipedia.org/wiki/Microelectrode_array). Chaque microélectrode est reliée électriquement par une connexion électrique (via, fil, etc.) à une électronique de conditionnement et d'adressage.

On trouve également des matrices de transistors, appelés MTA (acronyme de « multi-transistor-array » en anglais). Les transistors sont alors utilisés dans une configuration dite EOS (acronyme de « Electrolyte-oxyde-silicium » en anglais). Le document « Electrical imaging of neuronal activity by multi-transistor-array (MTA) recording at 7,8 µm resolution » de Lambacher et al., Applied Physics A., 2004 décrit ainsi une matrice de 16384 transistors à effet de champ fabriqués avec une technologie CMOS (acronyme de « complementary metal oxide silicon » en anglais) et recouverts d'une fine couche isolante de dioxyde de titane (TiO₂).

Le document US2015/261024 décrit un modulateur électro-optique.

Ces dispositifs d'imagerie reposant sur une approche électronique ont été optimisés pour atteindre des résolutions spatiales satisfaisantes (inférieures à 10 µm). Toutefois, leur champ d'analyse est limité à 1 mm² ou quelques mm², par exemple 3,5 mm², du fait de l'espace pris par les connexions électriques et les circuits électroniques.

Cette limitation empêche d'imager des populations de neurones de grande taille, ou de tester, sur un même dispositif et un même échantillon plusieurs conditions expérimentales (par exemple plusieurs médicaments) en parallèle.

Par ailleurs, ces dispositifs électroniques sont complexes et coûteux à fabriquer.

Il existe donc un besoin de dispositifs d'imagerie électrophysiologiques simples à fabriquer et permettant d'analyser des échantillons de grande taille.

Ce besoin existe pour l'étude de population de neurones, et plus largement pour l'étude d'échantillons comprenant au moins une source apte à générer un signal électrique.

### RESUME DE L'INVENTION

L'invention offre une solution au problème évoqué précédemment en proposant d'utiliser les capacités de transduction électro-optique d'un film PDLC (acronyme de « polymer-dispersed crystal liquide » en anglais).

Un premier aspect de l'invention concerne un dispositif d'imagerie de l'activité électrique d'un échantillon, comprenant :
- un film de polymère dans lequel sont dispersées des gouttelettes de cristal liquide, appelé film PDLC,
- un composant fluidique transparent dans lequel est aménagée au moins une cavité adaptée à contenir l'échantillon,
- une électrode de référence transparente,
le film PDLC étant agencé entre ladite au moins une cavité et l'électrode de référence et configuré pour convertir un champ électrique créé dans une région du film PDLC en réponse à un potentiel électrique généré par l'échantillon en une variation de transparence de ladite région du film PDLC.

Le terme « transparent » désigne un matériau ou un élément présentant un coefficient de transmission optique supérieur à 85% pour au moins une longueur d'onde de la bande spectrale 400-800 nm.

Selon l'invention, il suffit de trois éléments (un composant fluidique avec au moins une cavité, un film PDLC et une électrode de référence) pour obtenir un dispositif de visualisation spatio-temporelle de l'activité électrique d'un échantillon.

La cavité permet de positionner l'échantillon en regard du film PDLC et de l'électrode de référence. Elle est bien adaptée aux applications « in-vitro ».

L'électrode de référence est utilisée pour détecter des potentiels électriques générés dans une pluralité de régions de l'échantillon.

Le film PDLC est utilisé comme transducteur, pour convertir ces potentiels électriques détectés en une variation de l'état de transparence de régions du film PDLC correspondant à (c'est-à-dire en vis-à-vis de) la pluralité des régions de l'échantillon. Cette variation de transparence est mesurable par imagerie optique.

Le film PDLC permet une visualisation directe, sans utilisation de polariseur et d'analyseur agencés de part et d'autre du film. Cette absence d'éléments polarisants, outre qu'elle évite une potentielle atténuation du signal optique, contribue à la simplicité du dispositif d'imagerie.

Le film PDLC présente en outre des propriétés mécaniques (tenue, flexibilité) adaptables, qui facilitent son intégration dans le dispositif d'imagerie. Le dispositif d'imagerie, déjà simple dans sa structure, est ainsi commode à fabriquer.

Le film PDLC peut en outre être fabriqué facilement dans des dimensions aussi grandes que souhaitées, par exemple de 4 cm x 4 cm. Il présente par ailleurs des capacités de transduction passives, c'est-à-dire sans besoin d'électrodes de détection, de connexions électriques ni de circuits électroniques d'adressage qui habituellement limitent le nombre de canaux de mesure, ou la superficie de la surface sensible.

Ces avantages propres au film PDLC confèrent au dispositif d'imagerie selon l'invention, outre une grande simplicité, un champ d'analyse intrinsèque nettement supérieur à l'état de l'art. Le dispositif d'imagerie convient ainsi intrinsèquement à l'imagerie d'échantillons de grande taille, s'étendant sur des superficies supérieures à 1 cm².

Le dispositif selon le premier aspect de l'invention peut également présenter une ou plusieurs caractéristiques ci-dessous, considérées individuellement ou selon toutes les combinaisons techniquement possibles.

Le film PDLC est en outre configuré pour varier en transparence à des potentiels électriques dans la gamme 100 µV - 100 mV.

Ainsi, le dispositif d'imagerie est configuré pour détecter et convertir dans le domaine optique des signaux électriques dont l'amplitude est typique des potentiels extracellulaires générés par des cellules excitées, telles que des neurones.

Les gouttelettes de cristal liquide du film PDLC ont en moyenne un diamètre compris entre 1 µm et 10 µm, de préférence compris entre 1 µm et 5 µm.

Le diamètre des gouttelettes détermine la résolution spatiale du dispositif d'imagerie. Le diamètre des gouttelettes ainsi spécifié est inférieur à, ou dans la gamme de, la taille des cellules neuronales. Ainsi, le dispositif d'imagerie convient à l'imagerie de l'activité électrique d'un neurone individuel.

Le film PDLC présente un taux de charge du cristal liquide dans le polymère compris entre 10% et 60 % en masse.

Ainsi, la variation de transparence est obtenue en réponse à des champs électriques dans la gamme de 0,1 V.µm à 5 V.µm.

Les gouttelettes de cristal liquide sont dispersées dans le film PDLC de telle sorte qu'on trouve dans le film PDLC au moins cinq gouttelettes de cristal liquide dans un volume de 10 µm x 10 µm x 10 µm.

De préférence, les gouttelettes de cristal liquide sont dispersées de manière homogène dans le film de polymère. Le terme « homogène » signifie ici que le nombre de gouttelettes 110 de cristal liquide par unité de volume présente une variation inférieure à 5 % au sein du film PDLC. Ainsi, les capacités de transduction du film PDLC sont sensiblement les mêmes sur toute la superficie du film PDLC.

Par ailleurs, la quantité de gouttelettes de cristal liquide par unité de volume est suffisante pour qu'une variation de transparence d'au moins 30 % soit obtenue dans une région du film PDLC correspondant à une région électriquement active de superficie 10 µm x 10 µm.

Comme les neurones s'étendent sur une superficie de 10 µm x 10 µm, cette configuration du film PDLC assure qu'un neurone, quelle que soit sa position en regard du film PDLC (et de l'électrode de référence), est associé à une zone transductive du film PDLC (c'est-à-dire, une zone dont la transparence varie en réponse à un champ électrique).

Contrairement aux dispositifs de l'état de l'art basés sur des arrangements matriciels (d'électrodes de détection, de transistors, etc.), le dispositif d'imagerie est dépourvu de zone non sensible, c'est-à-dire, en vis-à-vis de laquelle l'activité électrique de l'échantillon ne peut être détectée. Le dispositif d'imagerie présente ainsi une meilleure efficacité de détection.

Les gouttelettes de cristal liquide comprennent de préférence des particules dopantes choisies parmi les particules suivantes : nanoparticules de colorants, nanoparticules d'or, nanoparticules d'argent, nanoparticules d'oxyde de zinc, nano-fils d'or, nano-fils d'argent.

Ainsi, le film PDLC est plus sensible et réagit à des champs électriques dans la gamme 2 V et 50 V.

Le composant fluidique comprend en outre des moyens de polarisation interagissant avec le volume intérieur de ladite au moins une cavité, pour polariser l'échantillon.

Ainsi, le dispositif est bien adapté à l'analyse d'un échantillon immergé dans une solution telle qu'une solution de culture, par exemple un média neuronal.

Les moyens de polarisation comprennent une électrode transparente, dite contre-électrode.

Lorsque le film PDLC réagit à un champ électrique inférieur à une polarisation de 0,1 V et que le composant fluidique est isolant, les moyens de polarisation et l'électrode de référence peuvent être reliés électriquement à la masse

Alternativement, les moyens de polarisation et l'électrode de référence sont reliés électriquement aux bornes d'un générateur de tension pour créer un champ électrique dans le film PDLC égal ou supérieur à un champ électrique seuil du film PDLC à partir duquel la variation de transparence du film PDLC est obtenue.

Ainsi, le champ électrique généré par l'échantillon s'ajoute au champ électrique seuil généré par le générateur de tension. Cette configuration permet de prendre en compte le fait que le film PDLC varie en transparence à partir d'un champ électrique seuil.

Ladite au moins une cavité s'étend jusqu'au film PDLC, le polymère du film PDLC étant un matériau adapté à recevoir l'échantillon, tel que le polydiméthylsiloxane (PDMS).

Le fait d'utiliser du PDMS comme polymère du film PDLC donne au film PDLC une rigidité suffisante pour que l'électrode de référence et le composant fluidique puisse être assemblés à partir du film PDLC. Ainsi, la fabrication du dispositif s'en trouve simplifiée.

Ladite au moins une cavité s'étend jusqu'à une couche transparente et isolante disposée entre le film PDLC et la cavité.

Un deuxième aspect de l'invention concerne un système d'imagerie de l'activité électrique d'un échantillon, comprenant :
- Un dispositif d'imagerie selon le premier aspect de l'invention,
- Une source de lumière apte à émettre une onde lumineuse,
- Un capteur d'images adapté pour former une image de la lumière transmise par le dispositif d'imagerie sous l'effet de l'activité électrique de l'échantillon,
Le dispositif d'imagerie étant disposé entre la source de lumière et le capteur d'images, la source de lumière étant en regard de l'un des éléments choisis parmi l'électrode transparente et la cavité du dispositif d'imagerie, et le capteur d'images étant disposé en regard de l'autre desdits éléments choisis.

Un troisième aspect de l'invention concerne un procédé d'analyse d'un échantillon à l'aide du dispositif d'imagerie selon le premier aspect de l'invention, comprenant les étapes de :
- Disposition de l'échantillon dans la cavité du composant fluidique du dispositif d'imagerie, ledit dispositif d'imagerie étant disposé entre une source de lumière et un capteur d'images,
- Illumination du dispositif d'imagerie et de l'échantillon par une onde lumineuse produite par la source de lumière, dite onde incidente, l'onde incidente se propageant à travers le dispositif d'imagerie jusqu'au capteur d'images,
- Détection optique de l'activité électrique de l'échantillon comprenant :
   - une étape d'acquisition, par le capteur d'images, d'une image représentative de l'onde lumineuse ayant été propagée à travers le dispositif d'imagerie, dite onde transmise,
   - une étape de détection, par un processeur communiquant avec le capteur d'images, d'une modification des propriétés de transmission du dispositif d'imagerie sous l'effet de l'activité électrique de l'échantillon, à partir de l'image préalablement acquise.

L'étape de détection de la modification des propriétés de transmission du dispositif d'imagerie peut comprendre les étapes suivantes :
- Détermination de caractéristiques de l'image acquise,
- Corrélation de ces caractéristiques déterminées avec le champ électrique généré par l'échantillon, à partir de caractéristiques de calibration établies en réalisant les étapes de disposition de l'échantillon, d'illumination et de détection optique à l'aide d'un échantillon étalon.

Un quatrième aspect de l'invention concerne un procédé de fabrication d'un dispositif d'imagerie de l'activité électrique d'un échantillon, comprenant les étapes suivantes :
- Fourniture d'un substrat sacrificiel,
- Fabrication d'un film de polymère dans lequel sont dispersées des gouttelettes de cristal liquide, appelé film PDLC, le film PDLC étant configuré pour convertir un champ électrique créé dans une région du film PDLC en réponse à un potentiel électrique généré par l'échantillon en une variation de transparence de ladite région du film PDLC,
- Fabrication, sur une première face du film PDLC opposée au substrat sacrificiel, d'une électrode de référence transparente,
- Retrait du substrat sacrificiel du film PDLC, de sorte à laisser libre une deuxième face du film PDLC opposée à la première face,
- Fourniture d'un composant fluidique transparent dans lequel est aménagée au moins une cavité adaptée à contenir l'échantillon,
- Assemblage du composant fluidique sur la deuxième face du film PDLC, de sorte que le film PDLC est agencé entre ladite au moins une cavité du composant fluidique et l'électrode de référence.

Un cinquième aspect de l'invention concerne un procédé de fabrication d'un dispositif d'imagerie de l'activité électrique d'un échantillon, comprenant les étapes suivantes :
- Fourniture d'un substrat transparent,
- Fabrication d'une électrode de référence transparente sur le substrat transparent,
- Fabrication, sur l'électrode de référence, d'un film de polymère dans lequel sont dispersées des gouttelettes de cristal liquide, appelé film PDLC, le film PDLC étant configuré pour convertir un champ électrique créé dans une région du film PDLC en réponse à un potentiel électrique généré par l'échantillon en une variation de transparence de ladite région du film PDLC,
- Fourniture d'un composant fluidique transparent dans lequel est aménagée au moins une cavité adaptée à contenir l'échantillon,
- Assemblage du composant fluidique sur la face du film PDLC opposée à l'électrode de référence, de sorte que le film PDLC est agencé entre ladite au moins une cavité du composant fluidique et l'électrode de référence.

L'invention et ses différentes applications seront mieux comprises à la lecture de la description qui suit et à l'examen des figures qui l'accompagnent.

### BREVE DESCRIPTION DES FIGURES

Les figures sont présentées à titre indicatif et nullement limitatif de l'invention.
- La figure 1 représente schématiquement en vue de coupe transversale un premier mode de réalisation d'un dispositif d'imagerie de l'activité électrique d'un échantillon,
- La figure 2 représente schématiquement en vue de coupe transversale un deuxième mode de réalisation du dispositif d'imagerie,
- La figure 3 représente schématiquement en vue de coupe transversale un troisième mode de réalisation du dispositif d'imagerie,
- La figure 4 illustre le dispositif d'imagerie de la figure 3 en utilisation avec un échantillon biologique,
- La figure 5 est une photographie d'un exemple de composant fluidique utilisé dans les dispositifs d'imagerie des figures 1 à 3,
- La figure 6 représente schématiquement en vue de coupe transversale un système d'imagerie utilisant le dispositif d'imagerie de la figure 3.
- La figure 7 est un schéma synoptique illustrant l'enchaînement des principales étapes d'un procédé d'analyse de l'activité électrique d'un échantillon,
- Les figures 8A à 8H représentent schématiquement en vue de coupe des étapes ou sous-étapes d'un procédé de fabrication du dispositif d'imagerie des figures 1 à 3, et
- Les figures 9A à 9F représentent schématiquement en vue de coupe des étapes ou sous-étapes d'un procédé de fabrication alternatif au procédé de fabrication des figures 8A à 8H.

Sauf précision contraire, un même élément apparaissant sur des figures différentes présente une référence unique.

### DESCRIPTION DETAILLEE

Comme indiqué précédemment, la présente invention concerne notamment un dispositif d'imagerie pour détecter et visualiser l'activité électrique d'un échantillon, par exemple d'un échantillon biologique tel qu'un tissu neuronal, ou des cellules neuronales en culture dans un milieu de culture.

Le dispositif d'imagerie présente avantageusement un grand champ d'analyse, supérieur à 1 cm², une continuité de détection sur tout ce champ d'analyse, et une résolution spatiale micrométrique, comprise entre 1 µm et 20 µm, correspondant à l'échelle d'un neurone individuel.

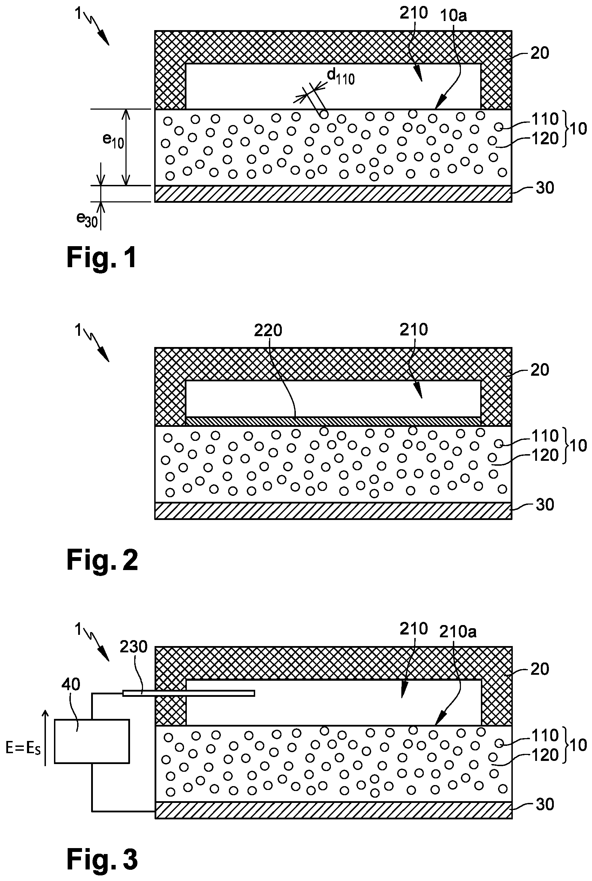

Un premier mode de réalisation et un deuxième mode de réalisation du dispositif d'imagerie sont représentés respectivement en figure 1 et en figure 2.

Un troisième mode de réalisation, compatible avec le premier ou le deuxième mode de réalisation, est représenté en figure 3.

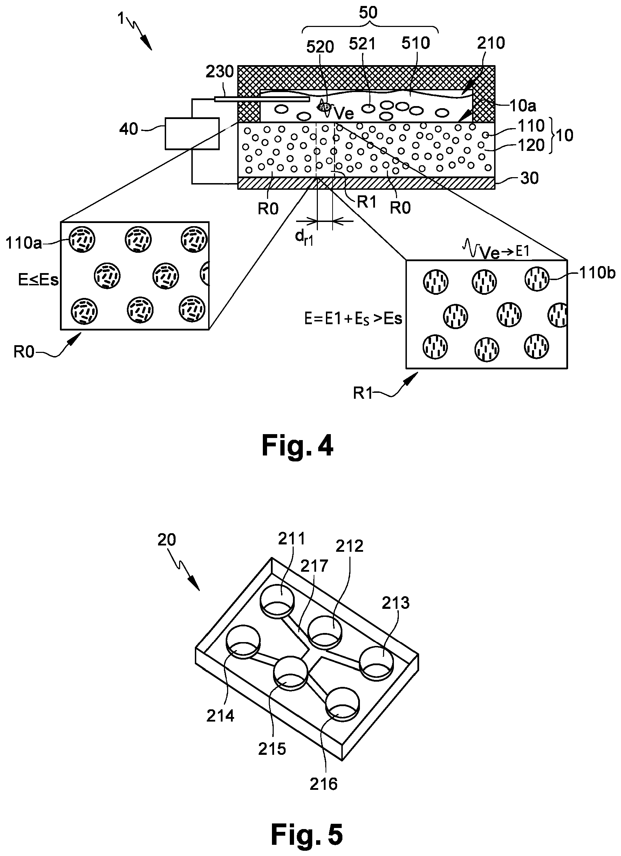

Ce troisième mode de réalisation est aussi représenté en figure 4 en utilisation avec un échantillon 50 de cellules neuronales 520, 521 immergées dans un milieu liquide 510 de culture, tel un média neuronal. Un exemple de média neuronal est une solution saline tamponnée au phosphate ou DBPS (acronyme de « Dubelcco's Phosphate Buffered Saline » en anglais).

De façon commune aux trois modes de réalisation, le dispositif 1 comprend :
- un film 10 de polymère 120 dans lequel sont dispersées des gouttelettes de cristal liquide 110, appelé film PDLC 10,
- un composant fluidique 20 transparent dans lequel est aménagée une ou plusieurs cavités 210 adaptées à contenir l'échantillon, et
- une électrode de référence 30 transparente.

Également de façon commune aux trois modes de réalisation, le film PDLC 10 est agencé entre la (ou les) cavité(s) 210 et l'électrode de référence 30. Ainsi qu'illustré en figure 4, le film PDLC 10 est configuré pour convertir un champ électrique E1 créé dans une région R1 du film PDLC 10 en réponse à un potentiel électrique Ve généré par l'échantillon 50 en une variation de transparence de ladite région R1 du film PDLC 10.

La variation de transparence est obtenue grâce aux propriétés électro-optiques intrinsèques du film PDLC 10. Celles-ci sont décrites ci-après.

De préférence, l'électrode de référence 30 est directement en contact direct avec le film PDLC 10. Elle peut être formée par dépôt d'une couche en un matériau conducteur et transparent sur le film PDLC 10.

Le matériau transparent et isolant est alors choisi en fonction de la méthode de dépôt pour être chimiquement compatible avec le film PDLC 10.

Dans une autre configuration, non représentée sur la figure 1, le dispositif d'imagerie 1 comprend en outre un substrat transparent sur lequel a été formée l'électrode de référence 30. L'électrode de référence 30 est alors agencée entre ledit substrat transparent et le film PDLC 10.

Le substrat transparent peut être en verre ou en un matériau polymère transparent choisi parmi les polymères suivants : polyéthylène téréphtalate (PET), polyéthylène naphtalate (PEN), polycarbonate (PC).

Lorsque le substrat transparent est en un polymère, l'électrode de référence 30 est avantageusement formée par un matériau conducteur et transparent choisi parmi les matériaux suivants : oxydes transparents conducteurs ou TCO (acronyme de « Transparent Conductive Oxydes » en anglais), dont oxyde de Zinc dopé aluminium (AZO), oxyde de Zinc (ZnO), ou par une encre conductrice transparente, comprenant par exemple des nanofils d'argent (Ag), d'or (Au) ou de poly(3,4-éthylènedioxythiophène) (PEDOT).

Lorsque le substrat transparent est en verre, l'électrode de référence 30 comprend une couche d'un matériau conducteur et transparent choisi parmi les matériaux suivants : oxyde d'indium et d'étain (ITO), oxyde de Zinc (ZNO), oxyde de Zinc dopé aluminium (AZO), oxyde d'étain (SnO2), argent (Ag), aluminium (AI), titane (Ti), cuivre (Cu), ou un empilement de couches formés par un ou plusieurs de ces matériaux.

L'épaisseur e₃₀ de l'électrode de référence 30 est de préférence comprise entre 10 µm et 2 mm.

Latéralement, l'électrode de référence 30 s'étend de préférence sur toute la surface du film PDLC 10.

Le composant fluidique 20 est formé d'un matériau transparent. Ce matériau est de préférence du polyméthylsiloxane ou PDMS. Ce matériau a en effet l'avantage d'être biocompatible, donc adapté à l'échantillon 50, et transparent.

Le composant fluidique 20 peut être formé d'un matériau qui est en outre électriquement isolant, tel que le PDMS, ou d'un matériau électriquement conducteur.

En référence à la figure 1, le composant fluidique 20 comprend une cavité 210. Comme mentionné précédemment, et en référence à la figure 4, cette cavité 210 est adaptée à contenir l'échantillon 50.

Les dimensions de la cavité 210 sont de préférence comprises entre 10 mm2 et 100 mm2.

Le composant fluidique 20 peut comprendre en outre au moins une entrée (non représentée en figure 4) reliée à la cavité 210 pour acheminer l'échantillon 50 dans la cavité 210. Cette entrée est par exemple équipée d'une valve, permettant d'assurer l'étanchéité fluidique entre la cavité 210 et l'extérieur du composant fluidique 20. La valve est de préférence formée d'un matériau transparent, par exemple de PDMS.

En référence à la figure 5, le composant fluidique 20 peut aussi comprendre plusieurs cavités, par exemple six cavités 211, 212, 213, 214, 215 et 216, reliées entre elles par des conduits 217 Dans le cas d'un échantillon biologique, ces conduits 217 sont adaptés aux solutions de culture 50.

Ces cavités ont de préférence des dimensions comprises entre 1x1 mm et 10x10 mm.

Chaque cavité 211-216 est alors en regard du film PDLC 10 et de l'électrode de référence 30.

Le film PDLC 10 est un film d'un matériau composite comprenant un matériau polymère et un cristal liquide. Ce matériau composite est formé par polymérisation d'un mélange d'une solution du polymère et d'une solution d'un matériau à cristal liquide (ou, dit plus simplement, d'un cristal liquide). Sous l'effet de la polymérisation, et en référence à la figure 1, le cristal liquide se trouve sous forme de gouttelettes 110 dispersées de manière discrète (séparées les unes des autres) dans le réseau du polymère 120.

Le film PDLC 10 a de préférence une épaisseur e₁₀ qui dépend de la taille des gouttelettes 110. L'épaisseur minimale peut être de 5 µm.

Latéralement, le film PDLC 10 peut s'étendre sur des dimensions aussi grandes que souhaitées. Par exemple, il s'étend sur des dimensions supérieures à 1 mm x 1 mm, de préférence supérieures à 1 cm x 1 cm, par exemple 4 cm x 4 cm.

Le film PDLC peut fonctionner dans un mode dit « normal », c'est-à-dire que le film PDLC 10 a la propriété de varier entre un état opaque ou semi-transparent (état off) et un état transparent (état on) après application d'un champ électrique perpendiculaire au plan du film PDLC 10. Toutefois, d'autres modes d'utilisation (inverse, etc.) peuvent être envisagés.

Dans le premier mode de réalisation représenté par la figure 1, la cavité 210 s'étend jusqu'au film PDLC 10. Autrement dit, elle débouche sur le film PDLC 10. Le polymère du film PDLC 10 est alors un matériau adapté à recevoir l'échantillon, tel que le polydiméthylsiloxane (PDMS). Le PDMS est avantageusement biocompatible, c'est-à-dire adapté à recevoir des échantillons biologiques.

Dans le deuxième mode de réalisation représenté par la figure 2, la cavité 210 ne s'étend pas jusqu'au film PDLC 10 mais jusqu'à une couche transparente et isolante 220, disposée entre le film PDLC 10 et la cavité 210. Cette couche transparente et isolante 220 fait de préférence partie du composant fluidique 20. L'avantage d'utiliser une telle couche intermédiaire est d'améliorer la tenue mécanique du dispositif d'imagerie 1. Ce mode de réalisation est privilégié lorsque le film a une rigidité insuffisante pour soutenir directement (c'est-à-dire via un contact direct) le composant fluidique 20.

Dans le troisième mode de réalisation représenté par la figure 3, le dispositif d'imagerie 1 comprend en outre une contre-électrode 230 utilisée comme moyens de polarisation, cette contre-électrode transparente 230 étant aménagée au moins en partie dans la cavité 210 pour polariser l'échantillon 50. La contre-électrode 230 est de préférence formée d'un matériau conducteur et transparent.

Par exemple, la contre-électrode 230 présente la forme d'une tige qui traverse une paroi du composant fluidique 20 pour déboucher à l'intérieur de la cavité 210.

Alternativement, la contre-électrode 230 peut avoir la forme d'une couche conductrice disposée sur une surface intérieure du composant fluidique 20 (délimitant la cavité 210), autre que le fond 210a (cf. figure 3) de la cavité 210, par exemple sur une des parois latérales de la cavité 210 ou sur la surface 10a du PDLC 10.

Une autre possibilité est que la contre-électrode 230 peut être formée d'une couche conductrice et transparente disposée sur le fond 210a de la cavité 210. Le fait qu'elle soit transparente permet à la lumière de traverser le dispositif d'imagerie 1, pour l'imagerie optique.

On notera que lorsque le composant fluidique 20 est formé d'un matériau conducteur, les parois et le fond 210a de la cavité 210 sont aussi conductrices. Dans ce cas, la cavité 210 forme intrinsèquement la contre-électrode 230.

Lorsque le composant fluidique est formé d'un matériau isolant et que la contre-électrode 230 n'est pas formée d'une couche conductrice et transparente disposée sur le fond 210a de la cavité 210, les moyens de polarisation de l'échantillon (comprenant la contre-électrode 230) et l'électrode de référence 30 peuvent être :
- soit reliées électriquement à la masse (configuration non représentée en figure 3) lorsque le film PDLC réagit à un champ électrique inférieur à une polarisation de 0,1 V,
- soit reliées électriquement aux bornes d'un générateur de tension 40 pour créer un champ électrique E dans le film PDLC égal ou supérieur à un champ électrique seuil Es du film PDLC à partir duquel la variation de transparence du film PDLC est obtenue. Ce champ électrique seuil Es correspond typiquement à une polarisation entre l'électrode de référence 30 et la contre-électrode 230 comprise entre 3 V et 5 V.

Lorsque le composant fluidique est formé d'un matériau conducteur ou que la contre-électrode 230 est formée d'une couche conductrice et transparente disposée sur le fond 210a de la cavité 210, les moyens de polarisation sont reliés électriquement aux bornes du générateur de tension 40.

Ainsi, les moyens de polarisation 230 permettent de configurer le champ électrique initial Es (c'est-à-dire en l'absence d'activité électrique de l'échantillon) dans le film PDLC 10.

Le principe de fonctionnement du dispositif d'imagerie 1 est décrit ci-après en relation avec la figure 4.

L'électrode de référence 30 agit comme moyens de génération d'un champ électrique E1 dans une région R1 du film PDLC 10 en réponse à l'activité électrique Ve d'un neurone 520.

Lorsque l'échantillon 50 n'est pas électriquement actif, un champ électrique Es préexiste dans le film PDLC 10, créé sous l'effet de la tension générée entre l'électrode de référence 30 et la contre-électrode 230 par le générateur de tension 40.

Lorsqu'une région 520 de l'échantillon 50 est électriquement active des charges s'accumulent au niveau de cette région active 520, générant un potentiel électrique Ve. Ce potentiel électrique Ve conduit à un champ E1 qui s'ajoute localement (c'est-à-dire dans la zone R1 de la région active 520) au champ électrique Es créé dans le film PDLC 10 par le générateur de tension 40. Le champ électrique résultant E est donc supérieur au champ électrique seuil Es.

Lorsqu'il n'y a pas de générateur de tension 40 et que l'électrode de référence 30 est portée à la masse, le principe de fonctionnement est identique, à la différence que le champ électrique préexistant dans le film PDLC 10 est nul.

Il convient de noter que le dispositif d'imagerie fonctionne de la même façon lorsqu'aucun moyen de polarisation n'est utilisé, comme dans le premier et deuxième modes de réalisation. Simplement, l'électrode de référence 30 est portée à la masse, et le champ électrique préexistant dépend de la polarisation de l'échantillon.

Le film PDLC sert de transducteur électro-optique pour convertir les potentiels électriques en une variation de la transparence du film PDLC dans la zone R1 de la région active 520. Dans les autres zones R0 du film PDLC 10, comme la tension entre l'électrode de référence 30 et l'échantillon 50 est inférieure ou égale à la tension de seuil Vs, la transparence n'a pas été modifiée par rapport à l'état off (le film est opaque ou semi-transparent dans ces zones R0).

La variation de transparence est liée aux propriétés de biréfringence des gouttelettes de cristal liquide 110.

Ainsi, en référence à la figure 4, lorsqu'aucun champ électrique n'est appliqué, ou lorsque celui-ci est inférieur au champ électrique Es seuil du film PDLC, les molécules de cristal liquide dans les gouttelettes 110a sont orientées aléatoirement les unes par rapport aux autres. Il y a donc des différences d'indices de réfraction entre chaque gouttelette 110a et le polymère 120, ce qui occasionne de la diffusion : la région R0 du film PDLC 10 apparaît opaque.

En revanche, lorsque le champ électrique E1 appliqué est supérieur au champ seuil du film PDLC 10, l'axe directeur de chaque gouttelette 110b s'aligne dans la même direction. Ainsi, l'indice de réfraction entre chaque gouttelette 110b est le même et la région R1 du film PDLC 10 apparaît transparente (disparition de la diffusion).

Les capacités de transduction du film PDLC 10 sont ainsi passives : il n'est pas besoin de circuits électroniques pour conditionner les potentiels électriques générés par l'échantillon ou les signaux de sortie du transducteur.

Le fait que le film PDLC 10 réagisse à des champs électriques et non à des courants électriques évite l'utilisation d'une contre-électrode qui serait disposée de l'autre côté du film PDLC 10 par rapport à l'électrode de référence 30, et qui aurait pour fonction d'amener les potentiels électriques de l'échantillon jusqu'au film PDLC 10.

Le film PDLC présente avantageusement une répartition homogène des gouttelettes de cristal liquide 110. Le terme « homogène » signifie ici que le nombre de gouttelettes 110 de cristal liquide par unité de volume présente une variation inférieure à 5 % au sein du film PDLC. Ainsi, les capacités de transduction du film PDLC sont sensiblement les mêmes sur toute la superficie du film PDLC. Dit autrement, les capacités de transduction du film sont spatialement régulières et continues.

De préférence, la répartition des gouttelettes est telle qu'on trouve au moins cinq gouttelettes de cristal liquide 110 dans un volume de 10 µm x 10 µm x 10 µm du film PDLC 10.

Comme les neurones s'étendent sur une superficie de 10 µm x 10 µm, cette configuration du film PDLC assure qu'un neurone, quelle que soit sa position en regard du film PDLC (et de l'électrode de référence), est associé à une zone transductive du film PDLC (c'est-à-dire une zone dont la transparence varie en réponse à un champ électrique). En référence à la figure 4, un neurone actif 520 est associé à une région R1 de transduction qui présente un diamètre dr1 égal au diamètre du neurone actif 520.

Plus généralement, la résolution spatiale n'est plus limitée, comme c'est le cas dans les dispositifs de l'état de l'art basés sur des transducteurs matriciels, par le pas de la matrice.

Les gouttelettes de cristal liquide 110 ont avantageusement un diamètre moyen d₁₁₀ compris entre 1 µm et 10 µm, de préférence compris entre 1 µm à 5 µm.

La résolution spatiale est ainsi adaptée à l'échelle d'un neurone, celui-ci ayant une taille de 15 µm.

Le polymère du film PDLC 10 est par exemple un polymère MOA-65 Norland ayant un indice réfraction égal à 1,524.

Le cristal liquide est un cristal liquide nématique. Il s'agit par exemple du cristal liquide MERCK MDA003969 présentant un indice de réfraction ordinaire nₒ1,498, un indice de réfraction extraordinaire nₑ 1,719, et une température isotropique de 106°C. Il présente une anisotropie d'indice Δ*_{ε}* égal à 2,7 à une fréquence supérieure ou égale à 50 kHz.

Par ailleurs, le taux de charge de cristal liquide dans le polymère peut être compris entre 10% et 60%.

Ainsi, la variation de transparence est obtenue en réponse à des champs électriques dans la gamme de 30 mV.µm à 100 mV.µm, qui correspond à la gamme des champs électriques générés par un échantillon biologique tel l'échantillon 50.

Les gouttelettes de cristal liquide peuvent comprendre des particules dopantes choisies parmi les particules suivantes : nanoparticules de colorants, nanoparticules d'or, nanoparticules d'argent, nanoparticules de ZnO, nano-fils d'or, nano-fils d'argent. Un procédé d'encapsulation de dopants dans des films PDLC est par exemple décrit dans le document « Preparation and electrooptic study of reverse mode polymer dispersed liquid crystal : performance augmentation with the doping of nanoparticles and dichroic dye », de Vandna Sharma et al., Journal of Applied Polymer Science, 48745, 2020.

Ces particules dopantes ont pour effet d'augmenter la sensibilité du film PDLC 10. Dit autrement, le film PDLC peut varier en transparence en réponse à des champs électriques d'une amplitude comprise entre 10 mV.µm et 1 V.µm couvrant ainsi la plage des champs électriques générés par les neurones. Comme par ailleurs la superficie du film PDLC 10 est centimétrique, on dispose d'un dispositif d'imagerie 1qui combine un grand champ d'analyse et une résolution spatiale micrométrique.

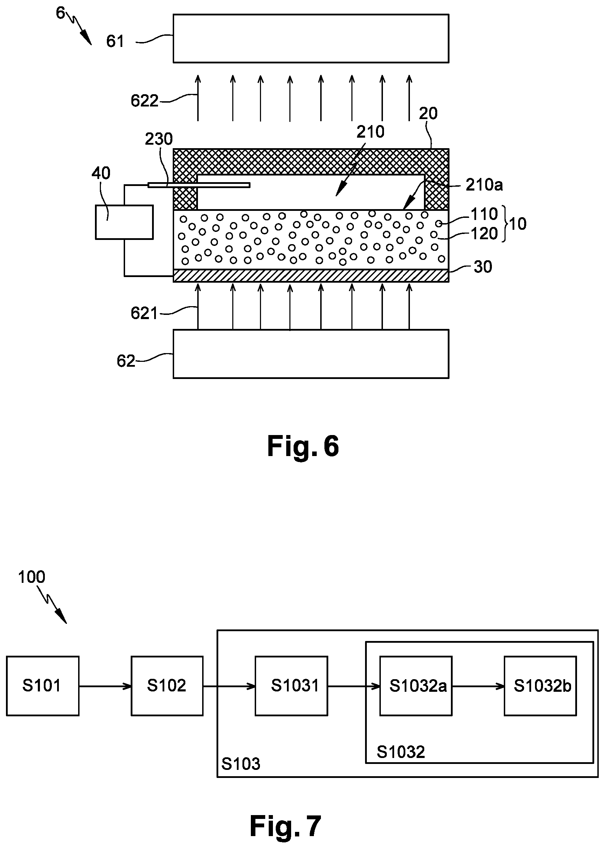

La figure 6 représente un système d'imagerie 6 de l'activité électrique d'un échantillon utilisant le dispositif d'imagerie 1 décrit précédemment.

En référence à la figure 6, le système d'imagerie 6 comprend :
- Le dispositif d'imagerie 1 selon l'un quelconque des modes de réalisation,
- Une source de lumière 62 disposée en regard de l'électrode de référence 30 et la cavité 210 du dispositif d'imagerie 1, adaptée pour émettre de la lumière 621 en direction du dispositif d'imagerie 1,
- Un capteur d'images 61 disposé en regard de la cavité 210, et adapté pour former une image de la lumière transmise 622 par le dispositif d'imagerie 1 sous l'effet de l'activité électrique de l'échantillon.

Le dispositif d'imagerie 1 est ainsi disposé entre la source de lumière 62 et le capteur d'images 61.

La source de lumière 62 est par exemple une source de lumière blanche. Elle peut comprendre des éléments optiques (polariseur, lentilles, fibres optiques, etc.) pour acheminer et/ou mettre en forme de la lumière 621 émise.

Le capteur d'images 61 est par exemple un capteur CCD (acronyme de « Charged Coupled Device » en anglais) intégré dans un microscope.

La source de lumière 62 et le capteur d'images 61 peuvent être interchangés par rapport au dispositif d'imagerie 1.

Ce système d'imagerie 6 permet d'imager de façon simple et directe l'activité électrique de l'échantillon.

Un procédé d'analyse 100 de l'échantillon 50 à l'aide du dispositif d'imagerie 1 est décrit ci-après en relation avec la figure 7.

Le procédé d'analyse 100 comprend les étapes principales suivantes :
- Disposition S101 de l'échantillon 50 dans la cavité 210 du composant fluidique 20 du dispositif d'imagerie 1, ledit dispositif d'imagerie 1 étant disposé entre une source de lumière et un capteur d'images tels que la source de lumière 62 et le capteur d'images 4 du système d'imagerie 6 décrit précédemment (cf. figure 6). L'échantillon est de préférence introduit dans la cavité une fois celle-ci assemblée sur l'ensemble formé par le film PDLC et l'électrode 30.
- Illumination S102 du dispositif d'imagerie 1 et de l'échantillon 50 par une onde lumineuse 622 produite par la source de lumière 62, dite onde incidente 622, l'onde incidente 621 se propageant à travers le dispositif d'imagerie 1 jusqu'au capteur d'images 61,
- Détection optique S103 de l'activité électrique de l'échantillon 50 comprenant :
   - une étape d'acquisition S1031, par le capteur d'images 61, d'une image représentative de l'onde lumineuse 622 ayant été propagée à travers le dispositif d'imagerie 1, dite onde transmise 621,
   - une étape de détection S1302, par un processeur communiquant avec le capteur d'images, d'une modification des propriétés de transmission du dispositif d'imagerie 1 sous l'effet de l'activité électrique des neurones 520,521 de l'échantillon, à partir de l'image préalablement acquise.

L'étape de détection S1032 de la modification des propriétés de transmission du dispositif d'imagerie 1 peut comprendre les étapes suivantes :
- Détermination S1032a de caractéristiques de l'image acquise, telles que régions d'intérêt, valeur moyenne des pixels dans cette région d'intérêt, etc.
- Corrélation S1032b de ces caractéristiques déterminées avec la localisation de la région électriquement active de l'échantillon 520 et du potentiel électrique généré par cette région 520, à partir de caractéristiques de calibration établies en réalisant les étapes S101, S102 et S103 de disposition de l'échantillon, d'illumination et de détection optique à l'aide d'un échantillon étalon.

Un procédé de fabrication du dispositif d'imagerie 1 est décrit ci-après. D'une manière générale, il comprend les étapes suivantes :
- Fabrication du film PDLC 10,
- Fourniture du composant fluidique 20,
- Fabrication de l'électrode de référence 30,
- Assemblage du film PDLC 10, de l'électrode de référence 30, et du composant fluidique 20 de sorte que le film PDLC 10 est agencé entre la ou les cavité(s) 210 du composant fluidique 20 et l'électrode de référence (30).

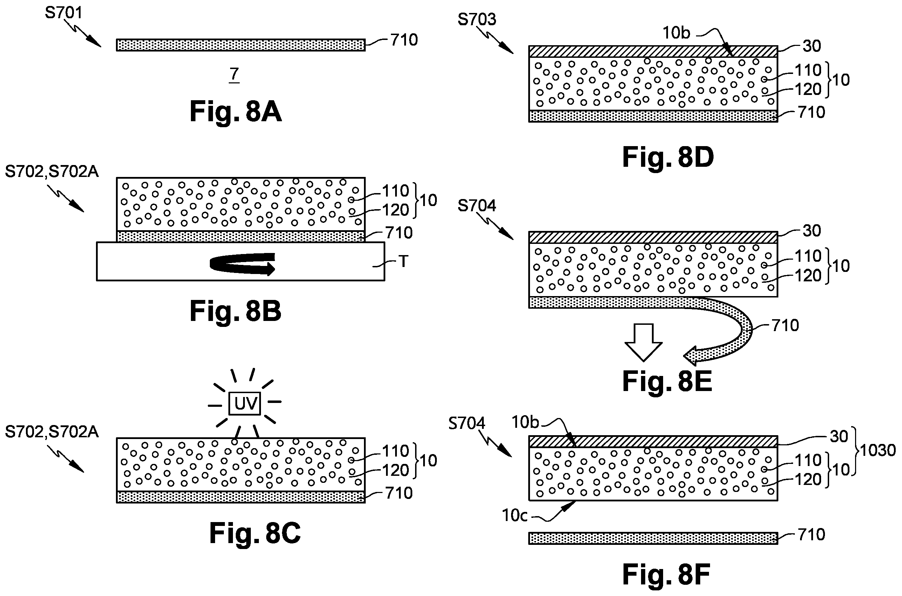

Un premier mode de mise en œuvre de ce procédé de fabrication est illustré sur les figures 8A à 8H.

Selon ce premier mode de mise en œuvre, le film PDLC 10 est d'abord fabriqué et sert de support à la fabrication de l'électrode de référence 30.

Selon ce premier mode de mise en œuvre, le procédé 7 débute avec l'étape S701, illustrée en figure 8A, de fourniture d'un substrat sacrificiel 710.

L'étape S701 se prolonge avec une étape S702 de fabrication du film PDLC 10 sur le substrat sacrificiel 710.

La fabrication comprend une première sous-étape S702A, illustrée en figure 8B de dépôt par centrifugation T (ou « spin-coating » en anglais) du mélange de la solution de polymère et de la solution de cristal liquide, et une deuxième sous-étape S702B, illustrée en figure 8C, de polymérisation, par exemple par exposition aux ultraviolets UV, du mélange déposé à l'étape S702A.

L'étape S702 est suivie d'une étape S703, illustrée en figure 8D, de formation de l'électrode de référence 30 sur une première face 10b du film PDLC opposée au substrat sacrificiel 710.

L'étape S703 se prolonge par une étape S704, illustrée en figure 8E, de retrait du substrat sacrificiel 710 du film PDLC 10.

En référence à la figure 8F, à l'issue de cette étape S704, une deuxième face 10c du film PDLC 10 opposée à la première face 10b est laissée libre, et le film PDLC 10 et l'électrode de référence 30 ont été assemblés pour former un ensemble 1030 film PDLC-électrode de référence.

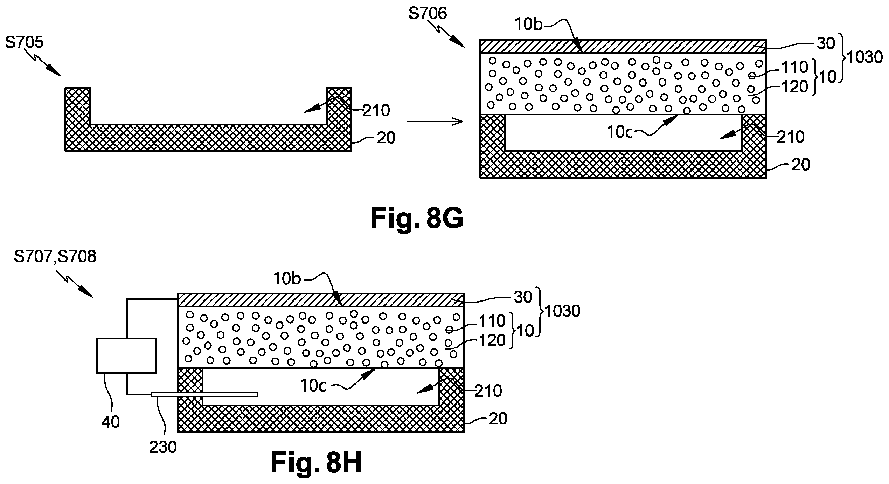

Les étapes S705 et S706 sont illustrées en figure 8G. L'étape S705 est une étape de fourniture du composant fluidique 20.

L'étape S706 consiste en l'assemblage du composant fluidique 20 sur la deuxième face 10c du film PDLC 10 de sorte que ladite cavité 210 fait face à l'électrode de référence 30, le film PDLC 10 étant alors agencé entre la cavité 210 et l'électrode de référence 30.

Deux étapes supplémentaires S707 et S708, illustrées en figure 8H, sont ensuite successivement effectuées pour, respectivement, réaliser les moyens de polarisation 230 du composant fluidique 20, et connecter électriquement ces moyens de polarisation. L'étape S706 consiste ainsi à insérer la contre-électrode 230 dans la cavité 210 à travers la paroi du composant fluidique 20. L'étape S707 consiste à fournir le générateur de tension 40 et à connecter électriquement ses bornes d'une part à l'électrode de référence 30 et d'autre part à la contre-électrode 230.

A l'issue de l'étape S707, l'ensemble 1030 film PDLC-électrode de référence et le composant fluidique 20 ont été assemblés, et le dispositif d'imagerie 1 est prêt à être utilisé.

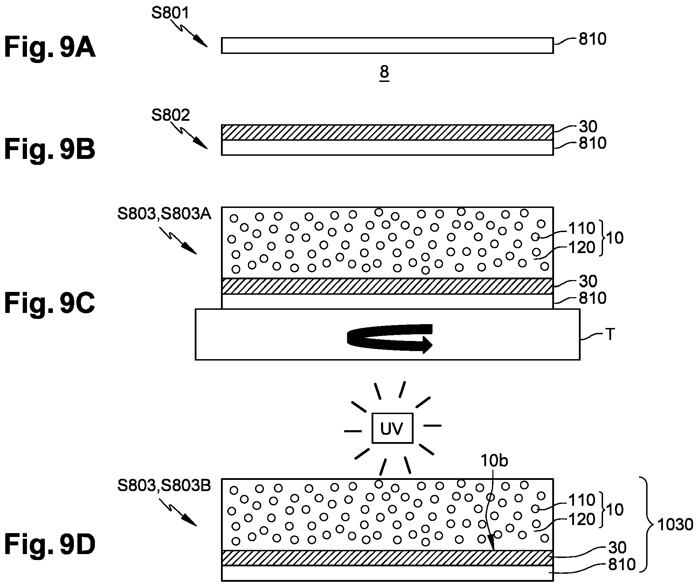

Un deuxième mode de mise en œuvre du procédé de fabrication est illustrésur les figures 9A à 9F.

Le procédé 8 selon ce deuxième mode de mise en œuvre diffère du premier mode de mise en œuvre en ce que l'électrode de référence 30 est formée sur un substrat transparent et sert de support pour fabriquer le film PDLC 10. Un tel mode de mise en œuvre sera privilégié lorsque la rigidité du film PDLC 10 est insuffisante pour servir de support à l'électrode de référence 30.

Le procédé 8 débute ainsi avec une étape S801, illustrée en figure 9A, de fourniture du substrat transparent 810.

Le procédé 8 se prolonge par une étape S802, illustrée en figure 9B, de fabrication, sur le substrat transparent 810, de l'électrode de référence 30.

Le procédé 8 se poursuit avec l'étape S803 consistant à fabriquer le film PDLC10 sur l'électrode de référence 30. L'électrode de référence est ainsi disposée sur la première face 10b du film PDLC 10.

Cette étape S803 comprend les sous-étapes S803A et S803B, illustrées respectivement sur la figure 9C et sur la figure 9D. Ces sous-étapes sont identiques aux sous-étapes S702A et S702B illustrées sur les figures 8B et 8C.

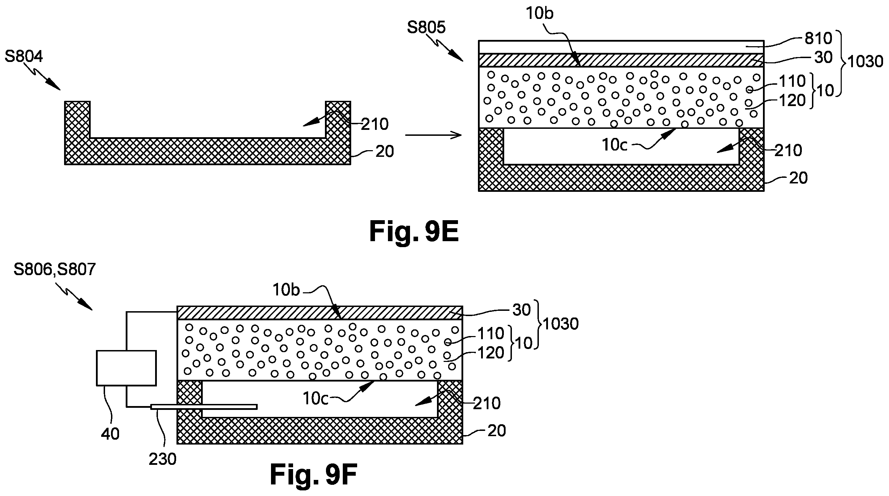

L'étape S803 est suivie des étapes S804 et S805, illustrés en figure 9E, et des étapes S806 et S807, illustrées en figure 9F. Ces étapes S804, S805, S806 et S807 sont identiques, respectivement, aux étapes S705, S706, S707 et S708 illustrées sur les figures 8G et 8H.

## Revendications

1. Dispositif d'imagerie (1) de l'activité électrique d'un échantillon (50), **caractérisé en ce qu'**il comprend :
- un film (10) de polymère (120) dans lequel sont dispersées des gouttelettes de cristal liquide (110), appelé film PDLC (10),
- un composant fluidique (20) transparent dans lequel est aménagée au moins une cavité (210) adaptée à contenir l'échantillon (50),
- une électrode de référence (30) transparente,
le film PDLC (10) étant agencé entre ladite au moins une cavité (210) et l'électrode de référence (30) et configuré pour convertir un champ électrique (E1) créé dans une région (R1) du film PDLC (10) en réponse à un potentiel électrique (Ve) généré par l'échantillon (50) en une variation de transparence de ladite région (R1) du film PDLC (10).

2. Dispositif d'imagerie (1) selon la revendication 1, dans lequel le film PDLC (10) est en outre configuré pour varier en transparence à des potentiels électriques (Ve) dans la gamme 100 µV - 100 mV.

3. Dispositif d'imagerie (1) selon l'une des revendications 1 à 2, dans lequel, les gouttelettes de cristal liquide (110) du film PDLC (10) ont en moyenne un diamètre (d₁₁₀) compris entre 1 µm et 10 µm, de préférence compris entre 1 µm et 5 µm.

4. Dispositif d'imagerie (1) selon l'une des revendications 1 à 3, dans lequel le film PDLC (10) présente un taux de charge de cristal liquide (110) dans le polymère (120) compris entre 10% et 60% en masse.

5. Dispositif d'imagerie (1) selon l'une des revendications 1 à 4, dans lequel les gouttelettes de cristal liquide (110) sont dispersées de manière homogène dans le film PDLC (10) de telle sorte qu'on trouve dans le film PDLC (10) au moins cinq gouttelettes de cristal liquide (110) dans un volume de 10 µm x 10 µm x 10 µm.

6. Dispositif d'imagerie (1) selon l'une des revendications 1 à 5, dans lequel les gouttelettes de cristal liquide (110) comprennent des particules dopantes choisies parmi les particules suivantes : nanoparticules de colorants, nanoparticules d'or, nanoparticules d'argent, nanoparticules de ZnO, nano-fils d'or, nano-fils d'argent.

7. Dispositif d'imagerie (1) selon l'une des revendications 1 à 6, dans lequel le composant fluidique (20) comprend en outre des moyens de polarisation (230) interagissant avec le volume intérieur de ladite au moins une cavité (210), pour polariser l'échantillon (50).

8. Dispositif d'imagerie (1) selon la revendication 7, dans lequel les moyens de polarisation (230) comprennent une électrode transparente (230), dite contre-électrode (230).

9. Dispositif d'imagerie (1) selon l'une des revendications 7 à 8, dans lequel les moyens de polarisation (230) et l'électrode de référence (30) sont reliés électriquement à la masse, le composant fluidique (20) étant formé d'un matériau électriquement isolant.

10. Dispositif d'imagerie (1) selon l'une des revendications 7 à 8, dans lequel les moyens de polarisation (230) et l'électrode de référence (30) sont reliées électriquement aux bornes d'un générateur de tension (40) pour créer un champ électrique dans le film PDLC égal ou supérieur à un champ électrique seuil (Es) du film PDLC (10) à partir duquel la variation de transparence du film PDLC (10) est obtenue.

11. Dispositif d'imagerie (1) selon l'une des revendications 1 à 10, dans lequel ladite au moins une cavité (210) s'étend jusqu'au film PDLC (10), le polymère (120) du film PDLC (10) étant un matériau adapté à recevoir l'échantillon (50), tel que le PDMS.

12. Dispositif d'imagerie (1) selon l'une des revendications 1 à 10, dans lequel ladite au moins une cavité (210) s'étend jusqu'à une couche transparente et isolante (220) disposée entre le film PDLC (10) et la cavité (210).

13. Système d'imagerie (6) de l'activité électrique d'un échantillon (50), comprenant :
- Un dispositif d'imagerie (1) selon les revendications 1 à 12,
- Une source de lumière (62) apte à émettre une onde lumineuse (621),
- **Un capteur** d'images (621) adapté pour former une image de la lumière transmise (622) par le dispositif d'imagerie (1) sous l'effet de l'activité électrique de l'échantillon (50),
le dispositif d'imagerie (1) étant disposé entre la source de lumière (62) et le capteur d'images (61), la source de lumière (61) étant en regard de l'un des éléments choisis parmi l'électrode de référence (30) et la cavité (210) du dispositif d'imagerie (1), et le capteur d'images (61) étant en regard de l'autre desdits éléments choisis.

14. Procédé d'analyse (100) d'un échantillon (50) à l'aide d'un dispositif d'imagerie (1) selon l'une des revendications 1 à 12, comprenant les étapes de :
- Disposition (S101) de l'échantillon (50) dans la cavité (210) du composant fluidique (20) du dispositif d'imagerie (1), ledit dispositif d'imagerie (1) étant disposé entre une source de lumière (62) et un capteur d'images (61),
- Illumination (S102) du dispositif d'imagerie (1) et de l'échantillon (50) par une onde lumineuse (622) produite par la source de lumière (62), dite onde incidente (622), l'onde incidente (622) se propageant à travers le dispositif d'imagerie (1) jusqu'au capteur d'images (61),
- Détection optique (S103) de l'activité électrique de l'échantillon (50) comprenant :
∘ une étape d'acquisition (S1031), par le capteur d'images (61), d'une image représentative de l'onde lumineuse ayant été propagée à travers le dispositif d'imagerie, dite onde transmise (621),
∘ une étape de détection (S1302), par un processeur communiquant avec le capteur d'images (61), d'une modification des propriétés de transmission du dispositif d'imagerie (1) sous l'effet de l'activité électrique de l'échantillon (50), à partir de l'image préalablement acquise.

15. Procédé d'analyse (100) selon la revendication 14, dans lequel l'étape de détection (S1032) de la modification des propriétés de transmission du dispositif d'imagerie (1) comprend les étapes suivantes :
- Détermination (S1032a) de caractéristiques de l'image acquise,
- Corrélation (S1032b) desdites caractéristiques déterminées avec le champ électrique (Ve) généré par l'échantillon (50), à partir de caractéristiques de calibration établies en réalisant les étapes de disposition (S101) de l'échantillon, d'illumination (S102) et de détection optique (S103) à l'aide d'un échantillon étalon.

16. Procédé (7) de fabrication d'un dispositif d'imagerie (1) de l'activité électrique d'un échantillon (50), comprenant les étapes suivantes :
- Fourniture (S701) d'un substrat sacrificiel (710),
- Fabrication (S702A, S702B) d'un film (10) de polymère (120) dans lequel sont dispersées des gouttelettes de cristal liquide (110), appelé film PDLC (10), le film PDLC (10) étant configuré pour convertir un champ électrique (E1) créé dans une région du film PDLC (10) en réponse à un potentiel électrique (Ve) généré par l'échantillon (50,520) en une variation de transparence de ladite région (R1) du film PDLC (10),
- Fabrication (S703), sur une première face (10b) du film PDLC (10) opposée au substrat sacrificiel (710), d'une électrode de référence (30) transparente,
- Retrait (S704) du substrat sacrificiel (710) du film PDLC (10), de sorte à laisser libre une deuxième face (10c) du film PDLC (10) opposée à la première face (10b),
- Fourniture (S705) d'un composant fluidique (20) transparent dans lequel est aménagée au moins une cavité (210) adaptée à contenir l'échantillon (50),
- Assemblage (S706, S707, S708) du composant fluidique (20) sur la deuxième face (10c) du film PDLC (10), de sorte que le film PDLC (10) est agencé entre ladite au moins une cavité (210) du composant fluidique (20) et l'électrode de référence (30).

17. 3Procédé (8) de fabrication d'un dispositif d'imagerie (1) de l'activité électrique d'un échantillon (50), comprenant les étapes suivantes :
- Fourniture (S801) d'un substrat transparent (810),
- Fabrication (S802) d'une électrode de référence (30) transparente sur le substrat transparent (810),
- Fabrication (S803A, S803B), sur l'électrode de référence (30), d'un film (10) de polymère (120) dans lequel sont dispersées des gouttelettes de cristal liquide (110), appelé film PDLC (10), le film PDLC (10) étant configuré pour convertir un champ électrique (E1) créé dans une région du film PDLC (10) en réponse à un potentiel électrique (Ve) généré par l'échantillon (50,520) en une variation de transparence de ladite région (R1) du film PDLC (10),
- Fourniture (S804) d'un composant fluidique (20) transparent dans lequel est aménagée au moins une cavité (210) adaptée à contenir l'échantillon (50),
- Assemblage (S805, S806, S807) du composant fluidique (20) sur la face (10c) du film PDLC (10) opposée à l'électrode de référence (30), de sorte que le film PDLC (10) est agencé entre ladite au moins une cavité (210) du composant fluidique (20) et l'électrode de référence (30).

## Patentansprüche

1. Bildgebungsvorrichtung (1) zur Darstellung der elektrischen Aktivität einer Probe (50), **dadurch gekennzeichnet, dass** sie umfasst:
- eine Folie (10) aus einem Polymer (120), in der Flüssigkristalltröpfchen (110) dispergiert sind und welche als PDLC-Folie (10) bezeichnet wird,
- eine transparente Fluidkomponente (20), in der mindestens ein Hohlraum (210) ausgebildet ist, der zur Aufnahme der Probe (50) geeignet ist,
- eine transparente Referenzelektrode (30),
wobei die PDLC-Folie (10) zwischen dem mindestens einen Hohlraum (210) und der Referenzelektrode (30) angeordnet und so gestaltet ist, dass sie ein in einem Bereich (R1) der PDLC-Folie (10) erzeugtes elektrisches Feld (E1) als Reaktion auf ein von der Probe (50) erzeugtes elektrisches Potential (Ve) in eine Änderung der Transparenz des genannten Bereichs (R1) der PDLC-Folie (10) umwandelt.

2. Bildgebungsvorrichtung (1) nach Anspruch 1, in der die PDLC-Folie (10) ferner so ausgelegt ist, dass sich ihre Transparenz bei elektrischen Potentialen (Ve) im Bereich von 100 µV bis 100 mV ändert.

3. Bildgebungsvorrichtung (1) nach einem der Ansprüche 1 bis 2, in der die Flüssigkristalltröpfchen (110) der PDLC-Folie (10) im Durchschnitt einen Durchmesser (d₁₁₀) zwischen 1 µm und 10 µm, vorzugsweise zwischen 1 µm und 5 µm, aufweisen.

4. Bildgebungsvorrichtung (1) nach einem der Ansprüche 1 bis 3, in der die PDLC-Folie (10) einen Füllgrad an Flüssigkristallen (110) im Polymer (120) zwischen 10 und 60 Massenprozent aufweist.

5. Bildgebungsvorrichtung (1) nach einem der Ansprüche 1 bis 4, in der die Flüssigkristalltröpfchen (110) homogen in der PDLC-Folie (10) dispergiert sind, so dass in der PDLC-Folie (10) pro 10 µm x 10 µm x 10 µm mindestens fünf Flüssigkristalltröpfchen (110) vorhanden sind.

6. Bildgebungsvorrichtung (1) nach einem der Ansprüche 1 bis 5, in der die Flüssigkristalltröpfchen (110) Dotierpartikel enthalten, die aus den folgenden Partikeln ausgewählt sind: Farbstoff-Nanopartikel, Gold-Nanopartikel, Silber-Nanopartikel, ZnO-Nanopartikel, Goldnanodrähte, Silbernanodrähte.

7. Bildgebungsvorrichtung (1) nach einem der Ansprüche 1 bis 6, in der die Fluidkomponente (20) ferner Polarisationsmittel (230) umfasst, die mit dem Innenvolumen des mindestens einen Hohlraums (210) in Wechselwirkung stehen, um die Probe (50) zu polarisieren.

8. Bildgebungsvorrichtung (1) nach Anspruch 7, in der die Polarisationsmittel (230) eine transparente Elektrode (230), eine sogenannte Gegenelektrode (230), umfassen.

9. Bildgebungsvorrichtung (1) nach einem der Ansprüche 7 bis 8, in der die Polarisationsmittel (230) und die Referenzelektrode (30) elektrisch geerdet sind, wobei die Fluidkomponente (20) aus einem elektrisch isolierenden Material gebildet ist.

10. Bildgebungsvorrichtung (1) nach einem der Ansprüche 7 bis 8, in der die Polarisationsmittel (230) und die Referenzelektrode (30) elektrisch an die Anschlüsse eines Spannungsgenerators (40) angeschlossen sind, um in der PDLC-Folie ein elektrisches Feld zu erzeugen, das gleich oder größer ist als ein Schwellenwert einer elektrischen Feldstärke (Es) der PDLC-Folie (10), ab dem die Änderung der Transparenz der PDLC-Folie (10) erzielt wird.

11. Bildgebungsvorrichtung (1) gemäß einem der Ansprüche 1 bis 10, in der sich der mindestens eine Hohlraum (210) bis zur PDLC-Folie (10) erstreckt, wobei das Polymer (120) der PDLC-Folie (10) ein Material ist, das zur Aufnahme der Probe (50) geeignet ist, wie beispielsweise PDMS.

12. Bildgebungsvorrichtung (1) nach einem der Ansprüche 1 bis 10, in der sich der mindestens eine Hohlraum (210) bis zu einer transparenten und isolierenden Schicht (220) erstreckt, die zwischen der PDLC-Folie (10) und dem Hohlraum (210) angeordnet ist.

13. Bildgebungssystem (6) zur Darstellung der elektrischen Aktivität einer Probe (50), umfassend:
- eine Bildgebungsvorrichtung (1) gemäß den Ansprüchen 1 bis 12,
- eine Lichtquelle (62), die eine Lichtwelle (621) aussenden kann,
- einen Bildsensor (621), der dazu ausgelegt ist, ein Bild des von der Bildgebungsvorrichtung (1) unter dem Einfluss der elektrischen Aktivität der Probe (50) durchgelassenen Lichts (622) zu erzeugen,
wobei die Bildgebungsvorrichtung (1) zwischen der Lichtquelle (62) und dem Bildsensor (61) angeordnet ist, wobei die Lichtquelle (62) einem der Elemente gegenüberliegt, die aus der Referenzelektrode (30) und dem Hohlraum (210) der Bildgebungsvorrichtung (1) ausgewählt sind, und der Bildsensor (61) dem anderen der ausgewählten Elemente gegenüberliegt.

14. Verfahren (100) zur Analyse einer Probe (50) unter Verwendung einer Bildgebungsvorrichtung (1) gemäß einem der Ansprüche 1 bis 12, das die folgenden Schritte umfasst:
- Anordnung (S101) der Probe (50) in dem Hohlraum (210) der Fluidkomponente (20) der Bildgebungsvorrichtung (1), wobei die Bildgebungsvorrichtung (1) zwischen einer Lichtquelle (62) und einem Bildsensor (61) angeordnet ist,
- Beleuchtung (S102) der Bildgebungsvorrichtung (1) und der Probe (50) durch eine von der Lichtquelle (62) erzeugte Lichtwelle (622), die als einfallende Welle (622) bezeichnet wird, wobei sich die einfallende Welle (622) durch die Bildgebungsvorrichtung (1) bis zum Bildsensor (61) ausbreitet,
- Optische Erfassung (S103) der elektrischen Aktivität der Probe (50), umfassend:
∘ einen Schritt (S1031) der Erfassung eines Bildes durch den Bildsensor (61), das die durch die Bildgebungsvorrichtung hindurchgeführte Lichtwelle darstellt, die als durchgelassene Welle (621) bezeichnet wird,
∘ einen Schritt (S1302) der Erfassung einer Veränderung der Übertragungseigenschaften der Bildgebungsvorrichtung (1) unter dem Einfluss der elektrischen Aktivität der Probe (50) durch einen mit dem Bildsensor (61) kommunizierenden Prozessor anhand des zuvor erfassten Bildes.

15. Analyseverfahren (100) nach Anspruch 14, in dem der Schritt der Erfassung (S1032) der Änderung der Übertragungseigenschaften der Bildgebungsvorrichtung (1) die folgenden Schritte umfasst:
- Bestimmung (S1032a) von Merkmalen des erfassten Bildes,
- Korrelation (S1032b) der ermittelten Merkmale mit dem von der Probe (50) erzeugten elektrischen Feld (Ve), ausgehend von Kalibrierungseigenschaften, die durch die Durchführung der Schritte der Anordnung (S101) der Probe, der Beleuchtung (S102) und der optischen Erfassung (S103) unter Verwendung einer Referenzprobe ermittelt wurden.

16. Verfahren (7) zur Herstellung einer Bildgebungsvorrichtung (1) der elektrischen Aktivität einer Probe (50), das die folgenden Schritte umfasst:
- Bereitstellen (S701) eines Opfersubstrats (710),
- Herstellung (S702A, S702B) einer Folie (10) aus einem Polymer (120), in der Flüssigkristalltröpfchen (110) dispergiert sind, als PDLC-Folie (10) bezeichnet, wobei die PDLC-Folie (10) so gestaltet ist, dass sie ein elektrisches Feld (E1), das in einem Bereich der PDLC-Folie (10) als Reaktion auf ein von der Probe (50, 520) erzeugtes elektrisches Potential (Ve) entsteht, in eine Änderung der Transparenz dieses Bereichs (R1) der PDLC-Folie (10) verwandelt,
- Herstellung (S703) einer transparenten Referenzelektrode (30) auf einer dem Opfersubstrat (710) gegenüberliegenden ersten Seite (10b) der PDLC-Folie (10),
- Entfernen (S704) des Opfersubstrats (710) von der PDLC-Folie (10), um eine der ersten Seite (10b) gegenüberliegende zweite Seite (10c) der PDLC-Folie (10) freizulegen,
- Bereitstellung (S705) einer transparenten Fluidkomponente (20), in der mindestens ein Hohlraum (210) ausgebildet ist, der zur Aufnahme der Probe (50) geeignet ist,
- Zusammenfügen (S706, S707, S708) der Fluidkomponente (20) auf der zweiten Seite (10c) der PDLC-Folie (10), so dass die PDLC-Folie (10) zwischen dem mindestens einen Hohlraum (210) der Fluidkomponente (20) und der Referenzelektrode (30) angeordnet ist.

17. Verfahren (8) zur Herstellung einer Bildgebungsvorrichtung (1) der elektrischen Aktivität einer Probe (50), das die folgenden Schritte umfasst:
- Bereitstellen (S801) eines transparenten Substrats (810),
- Herstellung (S802) einer transparenten Referenzelektrode (30) auf dem transparenten Substrat (810),
- Herstellung (S803A, S803B) auf der Referenzelektrode (30) einer Folie (10) aus einem Polymer (120), in der Flüssigkristalltröpfchen (110) dispergiert sind und die als PDLC-Folie (10) bezeichnet wird, wobei die PDLC-Folie (10) so gestaltet ist, dass sie ein elektrisches Feld (E1), das in einem Bereich der PDLC-Folie (10) als Reaktion auf ein von der Probe (50, 520) erzeugtes elektrisches Potential (Ve) entsteht, in eine Änderung der Transparenz dieses Bereichs (R1) der PDLC-Folie (10) verwandelt,
- Bereitstellen (S804) einer transparenten Fluidkomponente (20), in der mindestens ein Hohlraum (210) ausgebildet ist, der zur Aufnahme der Probe (50) geeignet ist,
- Zusammenfügen (S805, S806, S807) der Fluidkomponente (20) auf der der Referenzelektrode (30) gegenüberliegenden Seite (10c) der PDLC-Folie (10) in einer Weise, dass die PDLC-Folie (10) zwischen dem mindestens einen Hohlraum (210) der Fluidkomponente (20) und der Referenzelektrode (30) angeordnet ist.

## Claims

1. Device (1) for imaging electrical activity of a sample (50), **characterised in that** it comprises:
- a film (10) of polymer (120) in which droplets of liquid crystal (110) are dispersed, referred to as the PDLC film (10),
- a transparent fluidic component (20) wherein at least one cavity (210) adapted to contain the sample (50) is arranged,
- a transparent reference electrode (30),
the PDLC film (10) being arranged between said at least one cavity (210) and the reference electrode (30) and configured to convert an electric field (E1) created in a region (R1) of the PDLC film (10) in response to an electric potential (Ve) generated by the sample (50) into a variation in transparency of said region (R1) of the PDLC film (10).

2. Imaging device (1) according to claim 1, wherein the PDLC film (10) is further configured to vary in transparency at electric potentials (Ve) in the range 100 µV - 100 mV.

3. Imaging device (1) according to one of claims 1 to 2, wherein the liquid crystal droplets (110) of the PDLC film (10) have on average a diameter (d₁₁₀) of between 1 µm and 10 µm, preferably of between 1 µm and 5 µm.

4. Imaging device (1) according to one of claims 1 to 3, wherein the PDLC film (10) has a load rate of liquid crystal (110) in the polymer (120) of between 10% and 60% by mass.

5. Imaging device (1) according to one of claims 1 to 4, wherein the liquid crystal droplets (110) are homogeneously dispersed in the PDLC film (10) such that there are at least five liquid crystal droplets (110) in a volume of 10 µm x 10 µm x 10 µm in the PDLC film (10).

6. Imaging device (1) according to one of claims 1 to 5, wherein the liquid crystal droplets (110) comprise dopant particles selected from the following particles: dye nanoparticles, gold nanoparticles, silver nanoparticles, ZnO nanoparticles, gold nanowires, silver nanowires.

7. Imaging device (1) according to one of claims 1 to 6, wherein the fluidic component (20) further comprises polarisation means (230) interacting with the internal volume of said at least one cavity (210), to polarise the sample (50).

8. Imaging device (1) according to claim 7, wherein the polarisation means (230) comprise a transparent electrode (230), referred to as the counter-electrode (230).

9. Imaging device (1) according to one of claims 7 to 8, wherein the polarisation means (230) and the reference electrode (30) are electrically connected to the ground, the fluidic component (20) being formed of an electrically insulating material.

10. Imaging device (1) according to one of claims 7 to 8, wherein the polarisation means (230) and the reference electrode (30) are electrically connected to the terminals of a voltage generator (40) to create an electric field in the PDLC film equal to or greater than a threshold electric field (Es) of the PDLC film (10) from which the variation in transparency of the PDLC film (10) is achieved.

11. Imaging device (1) according to one of claims 1 to 10, wherein said at least one cavity (210) extends up to the PDLC film (10), the polymer (120) of the PDLC film (10) being a material adapted to receive the sample (50), such as PDMS.

12. Imaging device (1) according to any of claims 1 to 10, wherein said at least one cavity (210) extends to a transparent and insulating layer (220) disposed between the PDLC film (10) and the cavity (210).

13. System (6) for imaging electrical activity of a sample (50), comprising:
- An imaging device (1) according to claims 1 to 12,
- A light source (62) able to emit a light wave (621),
- an image sensor (621) adapted to form an image of the light transmitted (622) by the imaging device (1) under the effect of electrical activity of the sample (50),
the imaging device (1) being disposed between the light source (62) and the image sensor (61), the light source (61) facing one of the elements selected from the reference electrode (30) and the cavity (210) of the imaging device (1), and the image sensor (61) facing the other of said elements selected.

14. Method for analysing (100) a sample (50) using an imaging device (1) according to one of claims 1 to 12, comprising the steps of:
- Disposing (S101) the sample (50) in the cavity (210) of the fluidic component (20) of the imaging device (1), said imaging device (1) being disposed between a light source (62) and an image sensor (61),
- Illuminating (S102) the imaging device (1) and the sample (50) by a light wave (622) produced by the light source (62), referred to as the incident wave (622), the incident wave (622) propagating through the imaging device (1) to the image sensor (61),
- Optically detecting (S103) electrical activity of the sample (50) comprising:
∘ a step of acquiring (S1031), by the image sensor (61), an image representative of the light wave that has been propagated through the imaging device, referred to as the transmitted wave (621),
∘ a step of detecting (S1302), by a processor communicating with the image sensor (61), a modification in the transmission properties of the imaging device (1) under the effect of electrical activity of the sample (50), from the previously acquired image.

15. Analysis method (100) according to claim 14, wherein the step of detecting (S1032) the modification in the transmission properties of the imaging device (1) comprises the following steps of:
- Determining (S1032a) characteristics of the image acquired,
- Correlating (S1032b) said characteristics determined with the electric field (Ve) generated by the sample (50), from calibration characteristics established by carrying out the steps of disposing (S101) the sample, illuminating (S102) and optically detecting (S103) using a standard sample.

16. Method (7) for manufacturing a device (1) for imaging electrical activity of a sample (50), comprising the following steps of:
- Providing (S701) a sacrificial substrate (710),
- Manufacturing (S702A, S702B) a film (10) of polymer (120) in which droplets of liquid crystal (110) are dispersed, referred to as the PDLC film (10), the PDLC film (10) being configured to convert an electric field (E1) created in a region of the PDLC film (10) in response to an electric potential (Ve) generated by the sample (50, 520) into a variation in transparency of said region (R1) of the PDLC film (10),
- Manufacturing (S703), on a first face (10b) of the PDLC film (10) opposite to the sacrificial substrate (710), a transparent reference electrode (30),
- Removing (S704) the sacrificial substrate (710) from the PDLC film (10), so as to leave a second face (10c) of the PDLC film (10) opposite to the first face (10b) free,
- Providing (S705) a transparent fluidic component (20) in which at least one cavity (210) adapted to contain the sample (50) is arranged,
- Assembling (S706, S707, S708) the fluidic component (20) to the second face (10c) of the PDLC film (10), so that the PDLC film (10) is arranged between said at least one cavity (210) of the fluidic component (20) and the reference electrode (30).

17. Method (8) for manufacturing a device (1) for imaging electrical activity of a sample (50), comprising the following steps of:
- Providing (S801) a transparent substrate (810),
- Manufacturing (S802) a transparent reference electrode (30) on the transparent substrate (810),
- Manufacturing (S803A, S803B), on the reference electrode (30), a film (10) of polymer (120) in which droplets of liquid crystal (110) are dispersed, called the PDLC film (10), the PDLC film (10) being configured to convert an electric field (E1) created in a region of the PDLC film (10) in response to an electric potential (Ve) generated by the sample (50, 520) into a variation in transparency of said region (R1) of the PDLC film (10),
- Providing (S804) a transparent fluidic component (20) in which at least one cavity (210) adapted to contain the sample (50) is arranged,
- Assembling (S805, S806, S807) the fluidic component (20) on the face (10c) of the PDLC film (10) opposite to the reference electrode (30), so that the PDLC film (10) is arranged between said at least one cavity (210) of the fluidic component (20) and the reference electrode (30).
